Europäisches Patentamt

European Patent Office

Office européen des brevets

(19)

(11) Publication number: **0 127 291**
**B1**

(12) # EUROPEAN PATENT SPECIFICATION

(45) Date of publication of the patent specification:
11.03.87

(51) Int. Cl.⁴: **C 12 P 19/20, C 12 P 19/22**

(21) Application number: **84302289.8**

(22) Date of filing: **04.04.84**

(54) **Production of dextrose and maltose syrups using an enzyme derived from rice.**

(30) Priority: **20.05.83 US 496386**

(43) Date of publication of application:
**05.12.84 Bulletin 84/49**

(45) Publication of the grant of the patent:
**11.03.87 Bulletin 87/11**

(84) Designated Contracting States:
**DE FR GB**

(56) References cited:
**BE - A - 680 242**
**FR - A - 2 027 545**
**GB - A - 2 097 405**
**US - A - 4 355 110**

**AGRICULTURAL AND BIOLOGICAL CHEMISTRY, vol. 43, no. 1, 1979, pages 37-44; JIRO YAMADA et al.: "A debranching enzyme of rice seeds at milky stage, its purification and substrate specificities"**
**CHEMICAL ABSTRACTS, vol. 95, no. 12, December 1981, page 523, no. 202090b, Columbus, Ohio, US**

(73) Proprietor: **Miller Brewing Company, 3939 W. Highland Boulevard, Milwaukee Wisconsin 53223 (US)**

(72) Inventor: **Line, William F., 5200 S. Tuckaway Bivd. Greenfield, Wisconsin 53221 (US)**
Inventor: **Chaudhary, Vinod K., 9496 North Green Bay Road, Brown Deer Wisconsin 53029 (US)**
Inventor: **Chicoye, Etzer, 4374 N. Menomonee River Parkway Milwaukee, Wisconsin 53225 (US)**
Inventor: **Mizerak, Robert J., N8 30710 Cherokee Trail Waukesha, Wisconsin 53186 (US)**

(74) Representative: **Wynne-Jones, John Vaughan et al, Wynne-Jones, Lainé & James 22, Rodney Road, Cheltenham Gloucestershire GL50 1JJ (GB)**

## Description

The present invention relates generally to the field of making starch-derived dextrose or maltose syrups. More particularly, it relates to a method of preparing dextrose or maltose syrups from starch using a heat-stable debranching enzyme extracted from rice.

Prior to the advent of enzymatic saccharification, starch derived syrups were produced by straight acid hydrolysis. The main disadvantage of this process was that acid hydrolysis was non-specific and yielded syrups where the composition was: (1) dependent on the dextrose equivalent (D.E.) of the final syrup, and (2) fixed at any given D.E. These syrups would always contain dextrose, maltose, higher sugars (maltotriose, maltotetrose, etc.) and dextrins in certain proportions depending on the D.E. of the resultant syrup. Attempts to produce dextrose syrups by straight acid hydrolysis resulted in the production of unacceptable quantities of non-saccharide breakdown products, e.g., furfural. This problem was overcome when the starch industry abandoned non-specific acid saccharifications in favor of specific enzyme catalyzed saccharifications.

High dextrose syrups have been important to the starch derived syrup industry (hereafter industry) for some time. These syrups have been used as sweetening adjuncts in the formulation of various foods and beverages and more recently as the substrate stream for the production of high fructose corn syrups.

The discovery that glucoamylase could be used to saccharify starch to dextrose represented a significant breakthrough in the industry. Using this enzyme, the industry was able to: (1) minimize formation of sugars having a higher degree of polymerization (DP) and other by-products which were inherent with the acid process, and hence (2) produce syrups which contained 92-95% w/w dextrose while operating at a higher dissolved solids level than was possible using acid.

The production of high dextrose syrups by glucoseamylase is not without disadvantages. When a thinned starch hydrolizate of a low solids content is saccharified with a pure glucoamylase preparation, the dextrose syrup obtained normally has an extremely high dextrose content of 97% or higher. In addition, it is essentially free of non-dextrose saccharide impurities. Howerer, if the starch hydrolyzate solids are increased to the level economically required by the industry, the dextrose content of the resultant syrup substantially decreases and is accompanied by an increase in higher DP saccharide impurities. Nevertheless, industry had accepted a syrup of a lower dextrose content than desired in order to take advantage of the economics which are achieved by conducting the saccharification at the higher starch solids level.

In similar fashion, the industry began utilizing maltogenic enzymes, similar to malt diastase, to produce high maltose syrups. Malt diastase is capable of saccharifying starch to produce syrup containing from 50-55% maltose, at dissolved solids levels which are acceptable to the industry.

There are three basic classes of starch debranching enzymes. They are the glucoamylases, the isoamylases, and the pullulanases. Basically, pullulanases cleave the α-1,6 linkages of pullulan (an α-1,6 polymer of maltotriose isolated from a mold cell wall) to yield maltotriose. Pullulanases are specific for α-1,6 linkages and can debranch the α-1,6 linkages of starch to produce α-1,4 polysaccharides which can be converted by α-1,4 carbohydrases to sugars fermentable by brewer's yeast. Due to their effectiveness in hydroilyzing α-1,6 linkages, inclusion of a debranching enzyme in the saccharification increases either the yield (maltose) or rate of production and yield (glucose) of the desired product. Most commercial fungal glucóamylases do possess debranching activity (i.e., α-1,6 glycosidase activity), but at a much lower level than α-1,4 activity. Therefore, the inclusion of a pullulanase augments the relatively weak α-1,6 activity of glucoamylase, resulting in faster hydrolyisis of the α-1,6 linkages, and an increased rate of glucose production from the resulting α-1,4 polysaccharides.

In the commercial production of glucose using glucoamylase it is most common in the industry for economical reasons to use an acid or enzyme thinned starch hydrolyzate having a pH of about 4 to about 5 which is about the optimum for the glucoamylase and a temperature above 50°C. It would be advantageous to have a pullulanase that can operate efficiently under those pH and temperature conditions.

We have discovered that a debranching enzyme (pullulanase) extracted from rice can be used both in the preparation of high dextrose syrups under the most desirable commercial production conditions and in the production of high maltose syrups if the debranching enzyme is free of maltase and transglucosidase activities.

It is the primary object of the present invention to disclose a method of preparing high dextrose syrups under commercial pH and temperature conditions which employs both glucoamylase and a pullulanase derived from rice.

Further objects of the invention are to disclose enzyme preparations containing a debranching enzyme extracted from ungerminated or germinated rice in which the enzyme is free of contaminating maltase and transglucosidase activities, methods of preparing such enzyme preparations, and a method using the enzyme preparation in the production of high maltose syrups.

In the practice of the method of the present invention, a sugar syrup is prepared by saccharizing a thinned starch hydrolyzate at a temperature above about 45°C with an enzyme system which includes an α-1,4 carbohydrase and a heat-stable α-1,6 debranching enzyme (pullulanase) derived from rice is free of both maltase and transglucosidase activities.

The debranching enzyme from rice has significant advantages over the pullulanases previously used in that it effectively debranches in a pH range of about 4.0 to about 6.0 and has an optimum pH of about 5. Furthermore, it is surprisingly heat stable at about 45°C to about 60°C. Thus it can be used to prepare dextrose at a pH near the optimum for glucoamylase and under commercial temperature conditions.

The invention also relates to a method of obtaining enzyme preparations from rice in which the debranching enzyme is free of maltase and transglucosidase activities and methods of removing the contaminant activities from preparation containing the debranching enzyme.

The enzyme preparation containing the debranching enzyme or pullulanase, which is free of maltase and transglucosidase activities, is prepared as described below.

When seed or polished rice is the source, a pullulanase preparation may be obtained, which is free of the maltase and transglucosidase activities, by extracting the rice in an aqueous buffer having an ionic strengh $\leq$ .02M buffer salts; preferably between 0-.008M. When an extraction medium having an ionic strength much above .02M buffer salts is used, increasing quantities of the maltase and transglucosidase activities are extracted. In the preferred method, the rice is slurried in water at about 50°C for about 3 hours. The enzyme solution may be clarified by centrifugation, and the supernatant further purified by diafiltration and then concentrated to obtain an enzyme preparation containing the debranching enzyme free of the undesirable maltase and transglucosidase activities.

When malted rice is the source of the debrancher, the maltase and transglucosidase activities are coextracted with the pullulanase even when no buffer salts are added. Therefore, it is another objective of this invention to disclose methods of removing these contaminants from extracts of either polished or malted rice which contain them. The maltase and transglucosidase activities may be removed from pullulanase containing extracts by adsorbing one activity from another on weak ion exchangers. In one method, pullulanase is adsorbed on a weak anion exchanger, e.g. DEAE cellulose, under conditions which reject the components having maltase and transglucosidase activities. The pullulanase is then eluted from the resin with an ionic strength gradient. In the preferred method, the components having maltase and transglucosidase activities is adsorbed on a weak cation exchange resin, e.g. carboxymethylcellulose or IRC-50 under conditions which reject pullulanase (i.e. buffer salts not exceeding 0.05M and pH about 5 to about 7).

The debranching enzyme or pullulanase from rice, which has been extracted by the preferred method or otherwise freed of the contaminating carbohydrases, is added to thinned starch hydrolyzate in a reactor with a dextrose or maltose forming enzyme to form the desired sugar syrup. The effective amounts of the debranching and other enzyme to be added will depend upon the nature of the starch, the reaction conditions and the percentage of maltose or dextrose desired in the final syrup.

Normally, the debranching enzyme will pe present in an amount of at least about .05 units and preferably about .05 units to about .5 units of pullulanase activity per gran of dry substance (gds) of starch hydrolyzate and the $\alpha$-1,4 carbohydrase will be present in an amount which is effective to convert the $\alpha$-1,4 polysaccharides formed into the desired sugar syrup. Larger amounts of the debranching enzyme may be used but provide no particular advantage.

The $\alpha$-1,4 carbohydrase which is most commonly used in the industry when a high dextrose syrup is desired is a glucoamylase, such as that derived from *Aspergillus niger* which will cleave both $\alpha$-1,6 and 1,4 linkages. The combination of the rice debranching enzyme and the glucoamylase significantly reduces the time normally required to prepare a high dextrose syrup with glucoamylase alone. Although both of the enzymes possess $\alpha$-1,6 debranching activity, the rice enzyme is more potent than glucoamylase and as a result the reaction time is reduced. The amount of the rice debranching enzyme in such a mixture will normally be at least .05 units and preferably will be from about 0.15 to about 0.5 units pullulanase/gds and the amount of glucoamylase will be at least about .01 units and preferably about .15 units to about .3 units of glucoamylase activity per gds. The reaction may be conducted at the same pH and temperature at which commercial preparations are normally made, i.e. a pH of about 5 and a temperature of about 50°-60°C (e.g. 55°C). The use of commercial conditions is possible because the optimum pH of the rice enzyme is about 5 which is close to that of glucoamylase.

The $\alpha$-1,4 carbohydrase which is used when the desired product is a high maltose syrup is a maltose producing enzyme, such as a barley or malt diastase. The amount of enzyme to be added is preferably the minimum amount required to convert the $\alpha$-1,4 polysaccharides to maltose. Normally an amount of at least about 1.2 units of diastase/gds is used. Larger amounts can be used but are less economical. The reaction is preferably conducted at a pH of about 6 and a temperature of about 45°C to 60°C (e.g. 55°C).

The method of enzyme preparation described in US-A-4 355 110 is essentially identical to example 1 of the subject application and it is clear from table 1 that an enzyme so produced is *not* free of maltase and transglucosidase activity. As can be seen from tables 4 and 8 syrup prepared from the preparation of example 1 are even inferior to the control syrup which did not contain pullulanese. i.e. a preparation from the method described in No. 4 355 110 or in example 1 does not achieve the invention unless it is further treated as, for instance, in the method set out in example 3.

Accordingly it is obvious that the disclosure of U.S. Patent No. 4 355 110 does not anticipate either claim 1 or claim 7, because it is not free of maltase and transglucosidase activities. Further, the document does not in any way indicate the desirability of producing pullulanese which is free from maltase and transglucosidase nor does it in any way suggest how this might be achieved for the purposes of the invention.

The following analytical procedures were used in the examples described below:

*Materials and Methods*
    A.  Materials

Polished rice (#4 brewer's grade), a 10 D.E. acid thinned cornstarch, pullulan, Linter starch, maltose and glucoamylase.

B. Methods

1. Enzyme Assay

Pullulanase and amylase activities were monitored by the appearance of reducing sugars using 3,5 dinitrosalyçilic acid reagent. The following substrates were used to identify the two activities: (1) pullulanase-pullulan and (2) amylase-Linter starch. In all cases the enzyme was incubated with the 0.5% w/v substrate at pH 5.0 and 50°C. A unit of activity in both assays is defined as the appearence of 1 mg reducing sugar (calculated as maltose) per minute under these conditions.

Rice maltase was determined by the hydrolysis of 0.5% w/v maltose at pH 5.0 and 50°C. The glucose released was monitored and a unit of maltase activity defined as the production of one mg glucose per minute under these conditions.

2. Carbohydrate Analyses

The carbohydrate composition of the syrups were performed by high pressure liquid chromatography (HPLC) using either Q-15S, (7.8 × 600 mm, $Ca^{+2}$ form) resin or an HPX-87 prepacked column (7.8 × 300 mm, $Ca^{+2}$ form). The chromatograms were developed with $H_2O$ at a flow rate of 0.6 ml/min and a temperature of 85°C on a apparatus equipped with a refractive index detector and a microprocessor controlled programmer/data module.

Prior to analyses, the samples were: (1) diluted from 30° Brix (°B) to 10° B, and (2) heated to 100°C to inactivate the enzymes. The results are presented as percent total peak area (PTPA).

3. Other

All diafiltrations were performed on the DC-2 hollow fiber apparatus, equipped with a cartridge (M.W. cutoff = 10,000 daltons).

*Example 1*

*Extraction of Rice Pullulanase in High Salt Buffer*

An enzyme extract was prepared by doughing one hundred gm. floured polished rice into 200 ml 0.1M potassium phospate - 0.2M NaCl, pH 6.0 which had been attemperated to 50°C. The buffer also contained 0,007M sodium benzoate which was added as a bacteriostat. The suspension was stirred for 3 hr. at 50°C, after which it was clarified by centrifugation (10,000 rpm, 30 min, 5°C). The supernatant was then diafiltered vs. 3-4 volumes of extraction buffer and concentrated 3-4 fold prior to use.

Table 1 lists the pullulanase and maltase activities of this preparation [#1]. Specific activities of 1.07 u/mg protein and 1.89 u/mg protein were obtained vs. pullulan and maltose respectively. The ratio of maltase to pullulanase (M/P) was 1.77 for this preparation.

*Example 2*

*Maltase Concentration and Ionic Strength of the Extraction Medium*

Polished rice was extracted as described in Example 1 (i.e. at 50°C for 3 hrs.) except that the extraction medium was an aqueous solution of 0.007M sodium benzoate pH 6. After extraction, the supernatant was clarified by centrifugation, diafiltered vs. 3-4 volumes of extraction buffer, and concentrated 3-4 fold as above.

The results presented in Table 1 show that the pullulanase specific activity of the preparation thus obtained (Preparation 2) is about the same as that of the preparation of Example 1 but that the maltase specific activity has been reduced to 8% that of the preparation of Example 1. The M/P ratio of Preparation 2 was 0.12.

Increasing the ionic strength of the extraction buffer increased the amount of maltase extracted whereas the pullulanase activity remained fairly constant. Thus the M/P ratio increased as follows: (1) .02M acetate buffer, M/P = 0.33, (2) 0.5M acetate buffer, M/P = 1.28, and (3) 0.1M acetate buffer, M/P = 1.39. The latter two preparations contained about 79% and 82% as much maltase respectively as Preparation 1. From these data it was discovered that the concentration of buffer salts must be ≤ .02M in order to avoid extraction of the maltase and transglucosidase activities.

*Example 3*

*Adsorption on DEAE Cellulose*

The pullulanase preparation obtained in Example 1 (Preparation 1) was diafiltered vs. 4-5 volumes .02M phosphate buffer, pH 7.0, in preparation for treatment with DEAE cellulose, a weak anion exchanger. The resin was added at a rate of 21 mg (moist weight) per mg. protein and the resulting slurry was stirred for 1 hr. at room temperature. The resin, which had adsorbed the pullulanase activity quantitatively, was harvested by filtration. Pullulanase was eluated by stirring the resin under 0.1M phosphate buffer - 0.2M NaCl, pH 6.0 and the resin was separated from the pullulanase fraction by filtration.

The results summarized in Table 1 show that pullulanase Preparation 1 is essentially freed from maltase by adsorption to and elution from DEAE cellulose, (Preparation 3). Essentially all the maltase (>98%) was rejected from the resin. The pullulanase was purified about 7 fold by this treatment yielding a preparation of specific activity of 7.6 μ/mg protein with an M/P ratio of 0.04.

*Example 4*

*Extraction of Malted Rice and Removal of Maltase with Carboxymethyl Cellulose [CMC]*

Seed grade LaBelle rice was steeped in water at 22°C for 73 hrs. and then germinated for 5 days at the same temperature after which is was kilned at 50°C. Prior to extraction of the enzymes, the malted rice was floured and defatted with n-hexane to remove lipid.

The flour obtained was extracted under the same conditions as described for polished rice in Example 2. The extract was diafiltered in 4-5 volumes .02M phosphate buffer pH 7.0 in preparation for treatment with CMC. The resin was added at the same rate as described in Example 3 for DEAE cellulose. The slurry was stirred for 1 hr. at room temperature and the maltase free supernatant was harvested by filtration.

The activity profiles of these preparations, 4 and 5, are given in Table 2 along with that obtained from ungerminated polished rice. Comparing the malded rice extract 4 with the polished rice Preparation 2 several things are evident:

1. The amylase yield from the malted rice extract was increased about 20 fold over that obtained from ungerminated polished rice. The amylase/pullulanase (A/P) ratio of the enzyme extract from the malted rice was 6.1 as compared to an A/P of 0.3 obtained from Preparation 2.

2. The grain was sufficiently modified during germination so that a significant quantity of maltase was extracted even at low ionic strength. Thus the M/P was 0.76 for the malted rice extract 4 as compared to 0.12 for polished ungerminated rice 2 extracted under the same conditions.

3. The specific activity of the pullulanase (i.e., $\mu$/mg protein) was lower for Preparation 4 than for Preparation 2. This is due to the fact that the malted rice flour still contained the hull and bran which were removed when the ungerminated rice was polished. Hence considerably more structural protein was extracted from malted rice reducing the specific activity. Both grains yielded about the same quantity of pullulanase.

Treatment with CMC removed 77% of the maltase activity without adsorbing either the pullulanase or amylase. Thus, Preparation 5 was found to contain an M/P of 0.18 which compares favorably with the value of 0.12 obtained with Preparation 2. The A/P ratio remained uncharged.

The polished rice pullulanase preparations 1-3 and malted rice preparation 4 and 5 were used in the formulation of the syrups described below.

### Preparation of Syrups

All saccharification described below were conducted at an initial substrate concentration of 30% w/w. Typically to 150 g of an acid thinned 10 D.E. starch hydrolyzate was added 350 g deionized water. Sodium benzoate was added to a final concentration of 0.007M to prevent bacterial growth during saccharification and the pH of the solution was adjusted to the desired value with dilute NaOH or $H_2SO_4$. The final substrate concentration in all cases was 30° B ± 0.2° as determined by a hand held refractometer.

### Example 5

#### Preparation of High Dextrose Syrups

The dextrose saccharifications where carried out at 55°C at pH 5.0 using rice enzyme Preparation 1-3 in conjunction with glucoamylase. They were compared to a glucoamylase control run at its optimal pH of 4.3 at the same temperature. The enzyme addition rates are listed in Table 3 and are given in units per gm. dry substance (u/gds). In all saccharifications in which the rice enzyme extracts were used, the enzyme addition rate was adjusted to the desired pullulanase concentration. Thus the maltase level varied according to the preparation (i.e. lowest for Preparation 3 and highest for Preparation 1).

Table 4 lists the carbohydrate composition of syrups A-D with time. Note that the inclusion of any rice pullulanase preparation resulted in a much greater rate of hydrolysis of the dextrin (oligosaccharides > DP-3) fraction. Thus after 24 hrs., the level of this fraction ranged from 3-4.7% as compared to 13.8% of the control (syrup A). The control was found to contain greater concentrations of the higher oligosaccharides throughout the time course of these studies.

Despite the efficiency in reducing the dextrin fraction, inclusion of the rice enzyme Preparation 1 (Syrup B) in the saccharification yielded a syrup containing less dextrose than the control (syrup A). This was due to the production of DP-2 and DP-3 sugars, particularly the former. Thus after 72 hrs. syrup B was found to contain 4.4 times as much DP-2 as A (9.6 vs. 2.2%) and 3.8 times as much DP-3 (1.5 vs. 0.4%). As a result syrup B yielded only 87.5% dextrose as compared to 94.7% for the control.

These problems were eliminated by using either Preparation 2 or Preparation 3 both of which were free of transglucosidase and maltase activities. Preparations 2 and 3 yielded syrups (C and D, respectively) containing about 90% dextrose after 24 hrs. as compared to about 83% for the control.

The dextrin fraction was found to have been reduced to 3 and 5% in syrups C and D, respectively, as compared to 13.8% for syrup A. After 48 hrs. syrups C and D were found to contain 97.6 and 95.4% dextrose as compared to 91% for the control Syrup A. There was virtually no change for either of these syrups by extending hydrolysis another 24 hrs., whereas the control increased by 4% to 94.7 still 1.3 and 2.7% behind D and C, respectively. The DP-2 and DP-3 levels of syrups C and D were comparable to that found in syrup A and 3-4 fold less than syrup B prepared with the maltase containing enzyme Preparation 1.

Since it is less labor intensive to obtain a maltase free rice pullulanase preparation by reducing the ionic strength of the extraction media than by adsorbing the pullulanase on and eluting it from DEAE cellulose, the method by which Preparation 2 was prepared is preferred.

### Example 6

#### Preparation of Dextrose Syrups with Varying Concentrations of Rice Pullulanase

The experiment described in Example 5 was repeated using: (1) Preparation 2 containing the same level of rice pullulanase, 0.5 u/gds and, (2) 26% of this level, 0.13 u/gds. The results of this experiment are summarized in Table 5. Reduction of the pullulanase concentration decreased the rate at which the dextrin fraction was hydrolyzed. Thus after 17 hrs., Syrup G (0.13 u/dgs) contained about twice as much dextrin (8.3 vs. 4.1) as Syrup F. Both Syrups F and G contained less dextrin than the control Syrup E (13.8%). After 41 hrs. Syrups F and G contained 97.3% and 96.1% dextrose as compared to 92.3% for the control. After 63 hrs. the composition of Syrups F and G were identical (>97%) and both contained about 2.6% more dextrose than the control.

### Example 7

*Dextrose Syrups Made with Whole Rice in Conjunction with Glucoamylase*

Glucoamylase was added to the substrate solution described in Example 3 which had been attemperated to 60°C to yield a final concentration of 0.3 u/gds. Then #4 brewer's rice was added to a final concentration of 4.1% w/w (based on dry solids). Assuming an extraction efficiency of 2.3 u/gds rice, this yielded 0.1 u pullulanase/gds. The pH of the suspension was adjusted to 4.8 and it was stirred for 16 hrs. at 60°C. The rice was removed by filtration and the pH of the filtrate adjusted to 4.0. Saccharification was continued for 2 more days at 55°C. The carbohydrate composition of this syrup is given in Table 6. After 16 hrs., the dextrose content was about 93%, increasing to 96% after a total of 39 hrs. hydrolysis. No further change occurred after 26 hrs. more at 55°C. This syrup was comparable to Syrup D (Table 4) after 72 hrs. hydrolysis and contained about 1.3% more dextrose than either control Syrup A (Table 4) or Syrup E (Table 5). Increasing the ionic strength of the saccharification media (e.g. inclusion of buffer salts) resulted in syrups similar in composition to Syrup B (i.e. high DP-2, low dextrose).

### Example 8

*Use of Rice Pullulanase in Conjunction with Barley or Malt Diastase in the Production of High Maltose Syrups*

The maltose saccharifications were conducted in the same manner as described above Example 5 for dextrose except that the saccharification pH was 6.0. The diastase was extracted from either barley or malt by known methods.

Table 7 lists the enzyme addition rates to all the maltose syrups. Again, the rice pullulanase concentration was adjusted to a final concentration of 0.5 u/gds yielding maltase concentrations of 0.88 u/gds or 0.06 u/gds depending on whether Preparation 1 or Preparation 2 was used. The diastase level was adjusted to a final concentration of 1.2 u/dgs regardless of whether it was extracted from barley or malt.

The composition of syrups H-M is given in Table 8. The two controls, Syrup H (barley diastase) and Syrup I (malt diastase), yielded syrups containing 52 and 54% maltose and 37 and 29% dextrin, respectively, after 48 hours. The rice pullulanase Preparation 1, when used with either diastase (J and K) yielded syrups which contained high concentrations of dextrose and hence less maltose than the respective controls. The dextrose concentration increased with time and was generated by hydrolysis of maltose. Thus after 24 hrs. Syrup J contained 43.1% maltose and 14.2% dextrose. After 72 hrs. this syrup contained 21.8% dextrose and 36.6% maltose, i.e. the dextrose concentration increased by 7.6% while the maltose concentration decreased by 6.5%. The same trend is evident in Syrup K in which: (1) the dextrose concentration increased 5.3 and 8.6% from the 24 hr. level at 48 and 72 hrs., respectively, and (2) the maltose level decreased 4.6 and 7.7% over the same interval.

Inclusion of the maltase free rice pullulanase Preparation 2 (Syrups L and M): (1) largely eliminated the formation of dextrose, and (2) yielded syrups containing 13-15% more maltose than the two controls (Syrup H and I). Syrups L and M contained about 67% maltose after 24 hrs. hydrolysis, i.e. about 15-16% more than control Syrups H and I and 24% more than Syrups J and K in which Preparation 1 was used. Very little change occurred in the maltose concentrations by extendings the hydrolysis time to 48 hrs. Both syrups were found to contain 2-3% glucose, 17-20% DP-3, and 10-13% dextrin in addition to 67% maltose after 48 hrs. Syrup L contained 23% less dextrin than did Syrup H while Syrup M contained 18.3% less dextrin than did Syrup I after 48 hrs. The chief difference between using malt diastase (Syrup M) and barley diastase (Syrup L) is that the DP-3 level was increased and the dextrin fraction was decreased with malt diastase (Syrup M).

### Exemple 9

*Preparation of Maltose Syrups Using Malted Rice Extracts*

Table 9 lists the addition rates of the amylase and maltase activities when the pullulanase addition rate was fixed at 0.5 μ/gds. The only significant difference between Preparation 4 and Preparation 5 was that the maltase level was reduced over fourfold when Preparation 5 was used.

The carbohydrate compositions of syrups N and O are given in Table 10. The maltase free Preparation 5 yielded a syrup (O) that contained 67% maltose and 2.4% glucose after 24 hrs. Very little change occurred in the maltose concentration by extending the hydrolysis time to 41 hours. This syrup then compared favorably to syrups L and M produced using a maltase free ungerminated polished rice extract together with barley or malt diastase.

In contrast, the maltase containing Preparation 4 yielded a syrup (N) which contained less maltose and more glucose after 24 hours. Prolonged hydrolysis of this syrup resulted in a further production of glucose at the expense of maltose. Thus after 41 hrs. syrup N contained 2.8% less maltose and 2.6% more glucose.

The foregoing examples demonstrate that rice pullulanase is a useful adjunct in the preparation of: (1) dextrose syrups when used with glucoamylase, or (2) maltose syrups when used with either barley or malt diastase, providing care is taken to free the rice enzyme of the undesirable maltase and transglucosidase activities.

TABLE I

ENZYMATIC ACTIVITY PROFILE OF RICE PULLULANASE PREPARATIONS

| Prepara-tion # | Example # | Extraction Buffer | DEAE Treatment | Specific Activity | | |
|---|---|---|---|---|---|---|
| | | | | Pullulanase [P] | Maltase [M] | M/P |
| 1 | 1 | 0.1M Potassium Phosphate -0.2M NaCl, 0.007M Sodium Benzoate, pH 6.0 | — | 1.07 | 1.89 | 1.77 |
| 2 | 2 | 0.007M, Sodium Benzoate, pH 6.0 | — | 1.24 | 0.15 | 0.12 |
| 3 | 3 | Same as 1 | + | 7.64 | 0.33 | 0.04 |

TABLE 2

ENZYMATIC ACTIVITY PROFILE OF MALTED RICE PREPARATIONS

| Preparation # | Example # | Extraction Buffer | CMC Treatment | Specific Activity | | | |
|---|---|---|---|---|---|---|---|
| | | | | Pullulanase [P] | Amylase [A] | Maltase [M] | M/P |
| 4 | 4 | 0.007M Sodium Benzoate | — | 0.68 | 4.16 | 0.52 | 0.76 |
| 5 | 5 | 0.007M Sodium Benzoate | + | 0.73 | 4.73 | 0.13 | 0.18 |
| 2[1] | 2 | 0.007M Sodium Benzoate | — | 1.24 | 0.41 | 0.15 | 0.12 |

[1] Preparation 2 is the polished rice preparation described in Example 2.

TABLE 3

ENZYME ADDITION RATES FOR GLUCOSE SYRUPS

| Sirup # | Rice Preparation | Rice Extract Addition Rate u/gds | | Glucoamylase Addition Rate u/gds |
|---|---|---|---|---|
| | | Pullulanase | Maltase | |
| A | — | — | — | 0.15 |
| B | 1 | 0.50 | 0.88 | 0.15 |
| C | 2 | 0.50 | 0.06 | 0.15 |
| D | 3 | 0.50 | 0.02 | 0.15 |
| E | — | — | — | 0.15 |
| F | 2 | 0.50 | 0.06 | 0.15 |
| G | 2 | 0.13 | 0.015 | 0.15 |

TABLE 4

CARBOHYDRATE COMPOSITION OF GLUCOSE SYRUPS A-D

| Syrup # | Rice Pullulanase Preparation | Hydrolysis Time (hr.) | Sugar PTPA | | | |
|---|---|---|---|---|---|---|
| | | | Glucose | DP-2 | DP-3 | >DP-3 |
| A | — | 24 | 83.3 | 2.2 | 0.7 | 13.8 |
| B | 1 | 24 | 82.5 | 10.1 | 2.7 | 4.7 |
| C | 2 | 24 | 89.8 | 6.0 | 1.3 | 2.9 |
| D | 3 | 24 | 90.3 | 4.2 | 0.9 | 4.6 |
| A | — | 48 | 90.7 | 2.6 | 0.5 | 6.2 |
| B | 1 | 48 | 86.7 | 9.3 | 2.1 | 1.9 |
| C | 2 | 48 | 97.6 | 1.8 | 0.3 | 0.3 |
| D | 3 | 48 | 95.4 | 2.2 | 0.8 | 1.6 |
| A | — | 72 | 94.7 | 2.2 | 0.4 | 2.7 |
| B | 1 | 72 | 87.5 | 9.6 | 1.5 | 1.2 |
| C | 2 | 72 | 97.4 | 2.0 | 0.2 | 0.4 |
| D | 3 | 72 | 96.0 | 3.0 | 0.2 | 0.8 |

TABLE 5

CARBOHYDRATE COMPOSITION OF GLUCOSE SYRUPS E-G

| Syrup # | Rice Pullulanase Preparation/Level,u/gds | Hydrolysis Time (hr.) | Sugar PTPA | | | |
|---|---|---|---|---|---|---|
| | | | Glucose | DP-2 | DP-3 | >DP-3 |
| E | — | 17 | 83.8 | 2.0 | 0.4 | 13.8 |
| F | 2-0.5 | 17 | 90.1 | 5.0 | 0.8 | 4.1 |
| G | 2-0.13 | 17 | 87.2 | 3.7 | 0.8 | 8.3 |
| E | — | 41 | 92.3 | 1.6 | 0.4 | 5.7 |
| F | 2-0.5 | 41 | 97.3 | 1.8 | 0.4 | 0.5 |
| G | 2-0.13 | 41 | 96.1 | 1.7 | 0.3 | 1.9 |
| E | — | 63 | 94.9 | 1.7 | 0.3 | 3.1 |
| F | 2-0.5 | 63 | 97.6 | 2.2 | 0.2 | Trace |
| G | 2-0.13 | 63 | 97.4 | 1.8 | 0.3 | 0.5 |

TABLE 6

DEXTROSE SYRUP PREPARED WITH #4 BREWER'S RICE IN CONJUNCTION WITH GLUCOAMYLASE

| Total Hydrolysis Time (hrs.) | Glucose | Sugar PTPA | | |
|---|---|---|---|---|
| | | DP-2 | DP-3 | >DP-3 |
| 16 | 92.7 | 2.5 | 0.7 | 4.1 |
| 39 | 96.1 | 2.8 | 0.2 | 0.9 |
| 65 | 96.0 | 3.4 | 0.2 | 0.4 |

### TABLE 7

#### ENZYMES USED TO PRODUCE MALTOSE SYRUPS FROM MALTRIN M-100

| Syrup # | Rice Pullulanase Used | Rice Addition Rate u/gds | | Diastase Addition Rate u/gds | |
|---|---|---|---|---|---|
| | | Pullulanase | Maltase | Barley | Malt |
| H | — | — | — | 1.2 | — |
| I | — | — | — | — | 1.2 |
| J | 1 | 0.5 | 0.88 | 1.2 | — |
| K | 1 | 0.5 | 0.88 | — | 1.2 |
| L | 2 | 0.5 | 0.06 | 1.2 | — |
| M | 2 | 0.5 | 0.06 | — | 1.2 |

### TABLE 8

#### CARBOHYDRATE COMPOSITION OF SYRUPS H-M

| Syrup # | Rice Preparation Used | Diastase Used | Hydrolysis Time (hrs.) | Sugar PTPA | | | |
|---|---|---|---|---|---|---|---|
| | | | | DP-1 | Maltose | DP-3 | >DP-3 |
| H | — | Barley | 24 | 0.46 | 50.7 | 11.3 | 38.0 |
| I | — | Malt | 24 | 0.58 | 51.9 | 11.6 | 36.5 |
| J | 1 | Barley | 24 | 14.2 | 43.1 | 22.0 | 20.7 |
| K | 1 | Malt | 24 | 13.2 | 43.5 | 23.7 | 19.6 |
| L | 2 | Barley | 24 | 1.9 | 66.5 | 15.8 | 15.8 |
| M | 2 | Malt | 24 | 2.2 | 66.8 | 18.7 | 12.3 |
| H | — | Barley | 48 | 0.6 | 51.9 | 11.6 | 36.5 |
| I | — | Malt | 48 | 1.4 | 54.4 | 15.6 | 28.6 |
| J | 1 | Barley | 48 | — | — | — | — |
| K | 1 | Malt | 48 | 18.5 | 38.9 | 24.9 | 17.7 |
| L | 2 | Barley | 48 | 2.8 | 67.0 | 17.0 | 13.2 |
| M | 2 | Malt | 48 | 3.1 | 66.7 | 29.9 | 10.3 |
| J | 1 | Barley | 72 | 21.8 | 36.6 | 24.7 | 16.9 |
| K | 1 | Malt | 72 | 21.8 | 35.8 | 25.1 | 17.3 |

### TABLE 9

#### MALTED RICE ENZYME USED TO PRODUCE MALTOSE SYRUPS FROM M-100

| Syrup # | Malted Rice Preparation | Enzyme Level u/gds | | |
|---|---|---|---|---|
| | | Pullulanase | Amylase | Maltase |
| N | 4 | 0.50 | 3.06 | 0.38 |
| O | 5 | 0.50 | 3.24 | 0.09 |

### TABLE 10

#### CARBOHYDRATE PROFILES OF SYRUPS N AND O

| Syrup # | Malted Rice Preparation | Hydrolysis Time (hrs.) | Sugar PTPA | | | |
|---|---|---|---|---|---|---|
| | | | DP-1 | DP-2 | DP-3 | >DP-3 |
| N | 4 | 24 | 7.1 | 58.2 | 20.3 | 14.4 |
| O | 5 | 24 | 2.4 | 67.0 | 17.7 | 12.9 |
| N | 4 | 41 | 9.7 | 55.4 | 22.9 | 12.0 |
| O | 5 | 41 | 3.5 | 67.4 | 18.7 | 10.4 |

The rice which may be used as the source of the enzyme of the present invention is food-grade rice which has been treated at conditions mild enough to preserve the enzymatic activity. If ungerminated rice is used, either seed or polished dry milled rice may be used. Howerer, the preferred source is commercially polished dry milled brewer's rice because ease of procurement, higher specific activity yield and economy. The enzyme may be extracted from a wide variety of seed grade rice including LaBelle, LeBonnet, Nato, Starbonnet, or Brazos. LaBelle was chosen for the examples since it is the most plentiful domestic variety.

## Claims

1. A method of preparing a dextrose or maltose syrup from starch which comprises saccharifying a thinned starch hydrolyzate at a temperature above about 45°C with an enzyme system which includes an α-1,4 carbohydrase, and a heat-stable α-1,6 debranching enzyme (pullulanase) derived from rice, which is free of maltase and transglucosidase activities.

2. The method of claim 1 in which the debranching enzyme is added as an extract.

3. The method of claim 1 in which the α-1,4 carbohydrase is glucoamylase and the reaction is conducted at a pH of about 4.5 to about 5.5 and a temperature of about 50°C to about 60°C.

4. The method of claim 1 in which the α-1,4 carbohydrase is a maltose producing enzyme and the reaction is conducted at a pH of about 4.5 to about 7 and a temperature of about 50° to about 60°C.

5. The method of claim 1 in which both the α-1,6 debranching enzyme and the α-1,4 carbohydrase are derived from malted rice and the syrup is a maltose syrup.

6. The method of claim 1 in which the debranching enzyme is prepared from rice by suspending the rice in an extraction media which contains less than 0.02M salt at a temperature of 10°C to 60°C to obtain an extract containing the debranching enzyme free of maltase and transglucosidase activities.

7. The method of claim 1 in which the debranching enzyme is prepared by freeing rice pullulanase of undesired maltase and transglucosidase activities by treating the pullulanase with a cation exchange resin under conditions in which only the fraction containing maltase and transglucosidase activities is adsorbed.

8. The method of claim 1 in which the debranching enzyme is prepared by treating rice pullulanase with carboxymethyl cellulose which absorbs the maltase and transglucosidase activities.

## Patentansprüche

1. Verfahren zur Herstellung eines Dextrose- oder Maltosesirups aus Stärke, bei dem ein verdünntes Stärkehydrolysat bei einer Temperatur oberhalb etwa 45°C mit einem Enzymsystem verzuckert wird, das eine α-1,4 Carbohydrase und ein wärmebeständiges α-1,6 spaltendes Enzym (Pullulanase) enthält, das aus Reis gewonnen wird und keine Maltase- und Transglucosidaseaktivitäten enthält.

2. Verfahren nach Anspruch 1, dadurch gekennzeichnet, dass das spaltende Enzym als Extrakt zugesetzt wird.

3. Verfahren nach Anspruch 1, dadurch gekennzeichnet, dass die α-1,4 Carbohydrase Glucoamylase ist, und dass die Reaktion bei einem pH-Wert von etwa 4,5 bis 5,5 und bei einer Temperatur zwischen etwa 50 und 60°C durchgeführt wird.

4. Verfahren nach Anspruch 1, dadurch gekennzeichnet, dass die α-1,4 Carbohydrase ein Maltose erzeugendes Enzym ist und dass die Reaktion bei einem pH-Wert zwischen etwa 4,5 und 7 und bei einer Temperatur zwischen etwa 50 und 60°C durchgeführt wird.

5. Verfahren nach Anspruch 1, dadurch gekennzeichnet, dass sowohl das α-1,6 spaltende Enzym als auch die α-1,4 Carbohydrase aus gemalztem Reis hergestellt sind, und dass der Sirup ein Maltosesirup ist.

6. Verfahren nach Anspruch 1, dadurch gekennzeichnet, dass das spaltende Enzym aus Reis hergestellt wird, wobei der Reis bei einer Temperatur zwischen 10 und 60°C in einem Extraktionsmittel suspendiert wird, das weniger als 0,02 M Salz enthält, und ein Extrakt erhalten wird, der das spaltende Enzym frei von Maltase- und Transglucosidaseaktivitäten enthält.

7. Verfahren nach Anspruch 1, dadurch gekennzeichnet, dass das spaltende Enzym dadurch hergestellt wird, dass Reispullulanase von ungewünschten Maltase- und Transglucosidaseaktivitäten befreit wird, indem die Pullulanase mit einem Kationenaustauscherharz unter Bedingungen behandelt wird, bei denen nur die Fraktion, die Maltase- und Transglucosidaseaktivitäten enthält, absorbiert wird.

8. Verfahren nach Anspruch 1, dadurch gekennzeichnet, dass das spaltende Enzym dadurch hergestellt wird, dass Reispullulanase mit Carboxymethylcellulose behandelt wird, die die Maltose- und Transglucosidaseaktivitäten absorbiert.

## Revendications

1. Procédé de préparation d'un sirop de dextrose ou de maltose à partir de l'amidon, qui consiste à saccharifier un hydrolysat d'amidon dilué, à une température se situant au-dessus d'environ 45°C, avec un système enzymatique qui comprend une α-1,4 carbohydrase et une enzyme α-1,6 déramifiante (pullulanase), stable à la chaleur, dérivée du riz, qui est exempte des activités maltasique et gluocosidasique.

2. Procédé selon la revendication 1, dans lequel l'enzyme déramifiante est ajoutée sous la forme d'un extrait.

3. Procédé selon la revendication 1, dans lequel la α-1,4 carbohydrase est la glucoamylase et la réaction est conduite à un pH allant d'environ 4,5 à environ 5,5 et à une température allant d'environ 50° à environ 60°C.

4. Procédé selon la revendication 1, dans lequel la

α-1,4 carbohydrase est une enzyme productrice de maltose et la réaction est conduite à un pH allant d'environ 4,5 à environ 7 et à une température allant d'environ 50°C à environ 60°C.

5. Procédé selon la revendication 1, dans lequel à la fois l'enzyme α-1,6 déramifiante et l'α-1,4 carbohydrase sont dérivées du riz malté et le sirop est un sirop de maltose.

6. Procédé selon la revendication 1, dans lequel l'enzyme déramifiante est préparée à partir du riz par mise en suspension du riz dans un milieu d'extraction qui contient moins de 0,02M de sel à une température allant de 10°C à 60°C, afin d'obtenir un extract contenant l'enzyme déramifiante, exempte des activités maltasique et transglucosidasique.

7. Procédé selon la revendication 1, dans lequel on prépare l'enzyme déramifiante en débarrassant la pullulanase de riz des activités maltasique et transglucosidasique indésirées, par le traitement de la pullulanase avec une résine échangeuse de cations dans des conditions dans lesquelles seule la fraction contenant des activités maltasique et transglucosidasique est adsorbée.

8. Procédé selon la revendication 1, dans lequel l'enzyme déramifiante est préparée par traitement de la pullulanase de riz par de la carboxyméthyl cellulose qui absorbe les activités maltasique et transglucosidasique.